# EUROPEAN PATENT SPECIFICATION

(11) **EP 4 387 694 B1**
(45) Date of publication and mention of the grant of the patent: **01.10.2025**
(21) Application number: 22761476.5
(22) Date of filing: 03.08.2022
(51) Int. Cl.: A61M 11/00, A61M 15/00

(54) **MEDICAMENT DELIVERY DEVICE**
MEDIKAMENTENVERABREICHUNGSVORRICHTUNG
DISPOSITIFS D'ADMINISTRATION DE MÉDICAMENTS

(30) Priority: 17.08.2021 US 202163233803 P; 29.09.2021 EP 21199678
(43) Date of publication of application: 26.06.2024
(73) Proprietor: SHL Medical AG, 6302 Zug (CH)
(72) Inventor: SÄLL, Daniel, 131 28 Nacka Strand (SE)
(86) International application number: PCT/EP2022/071837
(87) International publication number: WO 2023/020842

(56) References cited:
- WO-A1-2019/170718
- US-A1- 2004 182 387
- US-A1- 2014 251 330
- US-A1- 2021 196 895

## Description

### TECHNICAL FIELD

The present disclosure relates to a medicament delivery device configured to eject a dose of medicament for delivery to a user.

### BACKGROUND

One type of medicament delivery device referred to as a soft mist inhaler (SMI) has the advantage of creating an aerosol that is easy for the patient to inhale compared to e.g. a pressurized metered dose inhaler (PMDI) or a powder inhaler.

Currently, there is no SMI on the market that provides a low cost option for everyday use. Prior art technologies commonly utilizes a technique referred to as impinging jets requiring a high pressure (>200 bar) to form the aerosol, which results in a high cost inhaler where a user must apply a high force to eject the aerosol. An example is shown in US2014/251330.

Rayleigh jet technique inhalers generally only require a pressure of around 20 bars to generate an aerosol, and may therefore be embodied in the form of a light and simple device. However, nozzle configuration of such inhalers is not suitable for pressures above 100 bar, so safety measures must be implemented to prevent nozzle membrane burst.

Further, pump design needs to be independent of user operation in terms of applying pressure such that the user operation does not alter the pressure generation during aerosol formation as no aerosol will be generated below 15 bars. There is thus room for improvement of prior art medicament delivery devices.

### SUMMARY

The invention is defined by the appended claims.

One objective is to solve, or at least mitigate, this problem in the art and thus provide an improved medicament delivery device.

This objective is attained in an aspect a medicament delivery device comprising a main body accommodating a medicament cartridge, a piston rod arranged at an outlet end of the medicament cartridge, the piston rod accommodating a channel an inlet end of which is arranged to be inserted in the medicament cartridge for access to and transport of the medicament, and a spring configured to spring load the piston rod such that the spring is compressed upon the piston rod being moved towards a distal end of the main body. The medicament delivery device further comprises a metering chamber configured to accommodate a dose of medicament being transported via the piston rod channel, a check valve arranged at an inlet end of the metering chamber, a medicament delivery member arranged at an outlet end of the metering chamber and configured to eject the dose of medicament in the metering chamber for delivery to the user, and a loading button being configured to be accessible from an exterior of the main body, which when operated by the user to move from a first position to a second position in a direction towards the distal end of the main body contacts the spring loaded piston rod and causes the spring loaded piston rod to move towards said distal end and the spring to compress, thereby causing the medicament to flow from the medicament cartridge via the channel into the metering chamber and which when operated by the user such that the spring loaded piston rod is released causes the spring to expand and the spring loaded piston rod to move away from said distal end and into the first position, which movement causes the dose of medicament to leave the metering chamber and flow through the medical delivery member for delivery to the user.

In an embodiment, the loading button is configured to be slidably arranged along a longitudinal axis of the medicament delivery device from the first position to the second position.

In an embodiment, the spring loaded piston rod is configured to move into locking engagement with the main body upon the user operating the loading button to move into the second position.

In an embodiment, the loading button is arranged with a locking member configured to move into engagement with a mating locking member of the main body for causing the locking engagement of the spring loaded piston rod and the main body.

In an embodiment, the locking member of the loading button and the mating locking member of the main body are configured such that an audible indication is provided to the user upon the two locking members moving into engagement.

In an embodiment, the locking member of the loading button and the mating locking member of the main body is configured such that a tactile indication is provided to the user upon the two locking members moving into engagement.

In an embodiment, the locking member of the loading button is configured to cause a temporary deformation of the main body upon moving into contact with said mating locking member before the mating locking member snaps into engagement with the locking member of the loading button to cause the audible and/or tactile indication.

In an embodiment, the main body is configured such that a user press onto the main body causes the mating locking member to snap out of engagement with the locking member of the loading button and release the spring loaded piston rod from the locking engagement with the main body.

In an embodiment, the loading button is arranged with a contacting member configured to move into contact with a mating contacting member of the main body, the two contacting members being configured to cause an audible and/or tactile indication to the user upon moving into contact.

In an embodiment, the spring loaded piston rod is arranged with a gripping member configured to move into a gripping engagement with the medicament cartridge upon the loading button initially being operated to move the spring loaded piston rod into the second position to cause an inlet end of the channel to penetrate a septum of the medicament cartridge.

In an embodiment, the medicament delivery device further comprises an air flow shaper arranged at an outlet end of the metering chamber.

In an embodiment, the medicament cartridge is arranged with a plunger movably attached to a bottom section the medicament cartridge which is configured to move with the medicament along a longitudinal axis of the medicament delivery device upon the medicament being transported from the medicament cartridge via the channel towards the metering chamber.

In an embodiment, the medicament delivery device further comprises a sealing member arranged around the channel in the spring loaded piston rod.

In an embodiment, the medicament delivery device further comprises a nozzle arranged at an outlet end of the metering chamber and comprising a membrane through which the medicament passes and which is configured to eject the medicament via the medicament delivery member as an aerosol for inhalation by the user.

In an embodiment, the medicament delivery device further comprises a medicament indicator configured to indicate amount of medicament remaining in the medicament cartridge.

In an embodiment, the medicament indicator comprises a window configured to allow the user to view the medicament in the medicament cartridge.

In an embodiment, the spring is configured to cause a pressure of 15-100 bar to be applied to the medicament transported to the medicament delivery member upon expanding.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated.

### BRIEF DESCRIPTION OF THE DRAWINGS

Aspects and embodiments are now described, by way of example, with reference to the accompanying drawings, in which:
Figure 1a shows a perspective view of a medicament delivery device according to an embodiment;
Figure 1b illustrates an embodiment of the inhaler of Figure 1a further comprising a medicament indicator;
Figure 2 shows the inhaler of Figure 1a in an exploded view;
Figure 3a shows a sectional view of the inhaler being in an initial state;
Figure 3b illustrates docking of a medicament cartridge;
Figure 3c illustrates button lock interaction during the docking of the cartridge;
Figure 3d illustrates completion of the docking of the cartridge;
Figures 3e and 3f illustrate performing of a first-time priming;
Figure 3g illustrates contacting members configured to provide a user with an audible/tactile indication according to an embodiment;
Figures 4a and 4b illustrate loading of medicament dose into metering chamber; and
Figures 4c and 4d illustrate releasing of medicament does from metering chamber.

### DETAILED DESCRIPTION

The aspects of the present disclosure will now be described more fully hereinafter with reference to the accompanying drawings, in which certain embodiments of the disclosure are shown.

These aspects may, however, be embodied in many different forms and should not be construed as limiting; rather, these embodiments are provided by way of example so that this disclosure will be thorough and complete, and to fully convey the scope of all aspects of invention to those skilled in the art. Like numbers refer to like elements throughout the description.

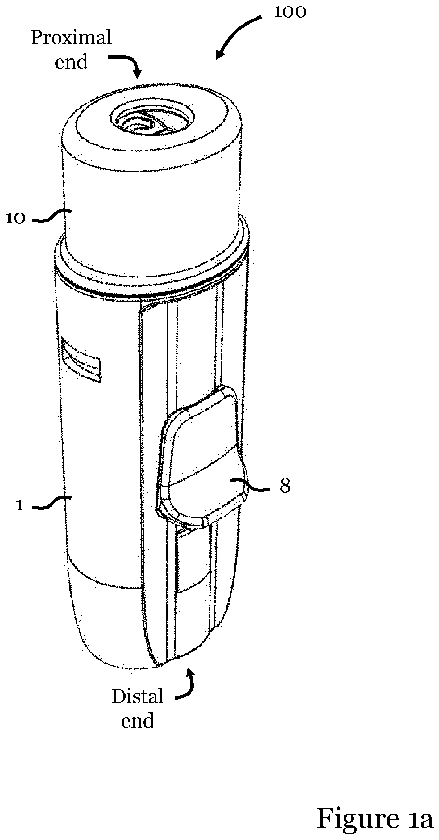

Figure 1a shows a perspective view of a medicament delivery device 100 according to an embodiment. The particular medicament delivery device 100 illustrated in Figure 1a is an inhaler configured to eject a dose of medicament for delivery to a user in the form of an aerosol via a medicament delivery member 10 commonly referred to as a mouthpiece. It is noted that embodiments are applicable to other medicament delivery devices delivering the medicament as a liquid jet.

An end of the inhaler 100 facing towards the user is referred to as proximal end while an end of the inhaler 100 facing away from the user is referred to as distal end.

The inhaler 100 further comprises a loading button 8 operated by the user in order to cause the inhaler 100 to eject a medicament dose from a medicament cartridge (not shown) being accommodated inside the main body 1.

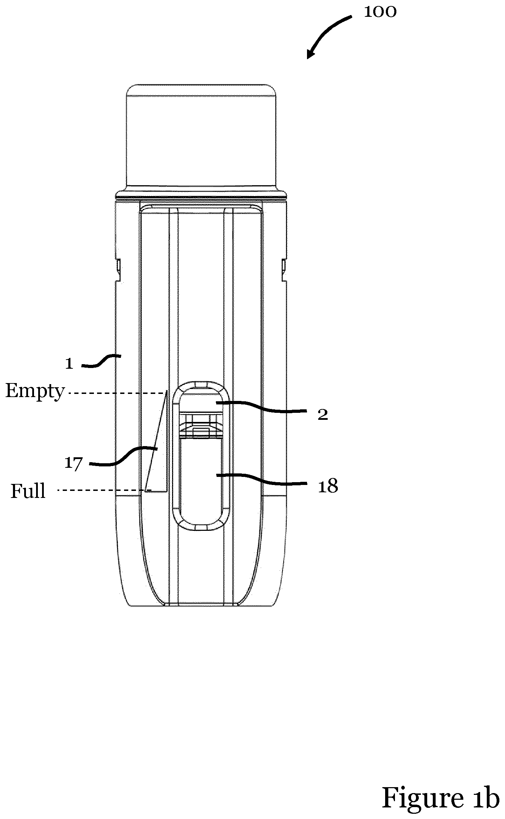

Figure 1b illustrates an embodiment of the inhaler 100 of Figure 1a where the main body 1 comprises a medicament indicator 17 configured to indicate amount of medicament remaining in the medicament cartridge 2 accommodated in the main body (in this embodiment arranged on the opposite side of the main body 1 with respect to the loading button). The indicator 17 may further be complemented with a window 18 to allow the user to view the medicament in the medicament cartridge, for instance to inspect a colour change or if any particles are present in the medicament.

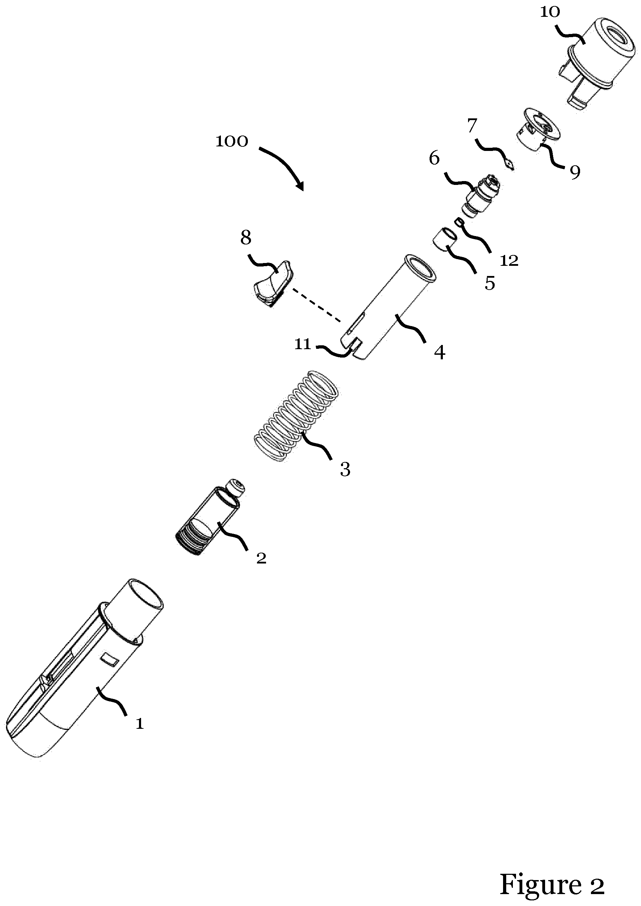

Figure 2 shows the inhaler 100 of Figure 1a in an exploded view. As can be seen, the main body 1 accommodates a medicament cartridge 2 and the loading button 8 is slideably arranged along a longitudinal axis of the main body 1 and thus along the inhaler 100.

The inhaler further comprises a piston rod 4 arranged at an outlet end of the medicament cartridge 2 (upon the cartridge 2 being accommodated inside the main body 1). The piston rod 4 accommodates a channel 11, an inlet end of which is arranged to be inserted in the medicament cartridge 2 for access to and transport of the medicament towards the proximal end of the inhaler 100.

Moreover, the inhaler 100 comprises a spring 3 configured to spring load the piston rod 4 such that the spring 3 is compressed upon the piston rod 4 being moved in a direction towards a distal end of the main body 1, which is being caused by a user operating the loading button 8 to slide along the main body 1 towards the distal end of the inhaler 100 is will be described.

The inhaler 100 also comprises a metering chamber 6 configured to accommodate a dose of medicament being transported from the cartridge 2 via the piston rod channel 11, and a check valve 12 arranged at an inlet end of the metering chamber 4 in order to prevent the medicament from flowing back from the chamber 4 into the channel 11.

Now, as will be described in great detail subsequently, upon the user operating the loading button 8 to move from a first position to a second position in a direction towards the distal end of the main body 1, the loading button 8 which is in contact with the spring loaded piston rod 4 will pull the rod 4 towards the distal end causing the spring 3 to compress, and the medicament will flow from the medicament cartridge 2 via the channel 11 into the metering chamber 6.

Subsequently, upon the user operating the loading button 8 such that the spring loaded piston rod 4 is released, the spring 3 will return to its expanded state which causes the spring loaded piston rod 4 to move away from the distal end and back into the first position. This movement causes the dose of medicament to exit the metering chamber 6 and flow through the mouthpiece 10 for delivery to the user in aerosol form.

In an optional embodiment, the inhaler 100 comprises a nozzle 7, for instance of a microelectromechanical systems (MEMS) type, which when being mounted inside the inhalator 100 is located at an outlet end of the metering chamber 6 and comprises a membrane configured to enable formation of the medication aerosol being ejected from the mouthpiece 10 for inhalation by the user.

Further optionally the inhaler 100 comprises an air flow shaper 9 arranged at an outlet end of the metering chamber 6 configured to facilitating shaping of inhalation air to prevent droplet coalescence and to create a sufficient inhalation air resistance, and a sealing member 5 arranged around the channel 11 in the spring loaded piston rod 4 at the inlet of the metering chamber 6 to prevent medicament leakage.

Now, the inhalator 100 is typically delivered with the medicament cartridge 2 already being mounted (and being intact) inside the main body 1 and thus a lid (commonly referred to as septum) of the cartridge 2 must be penetrated by the sharp inlet end of the channel 11 for the inhaler 100 to be able to deliver the medicament to a user.

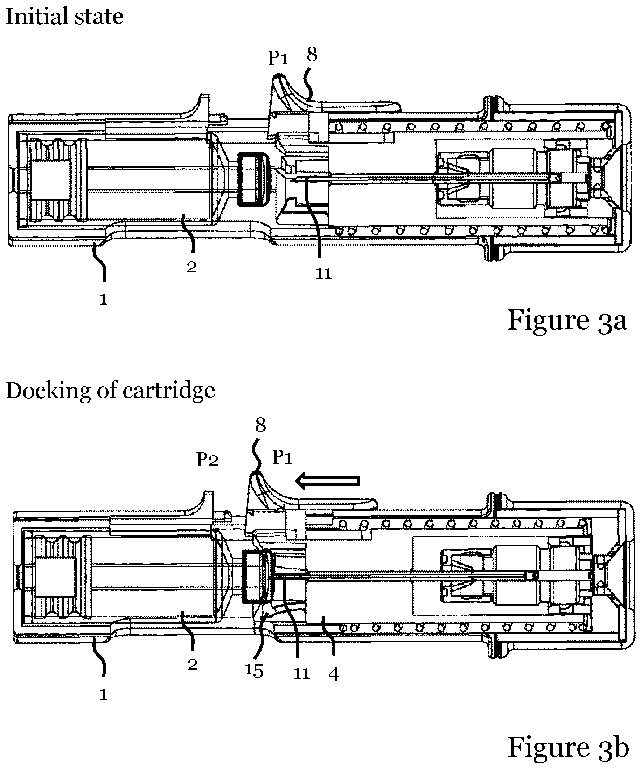

Figure 3a shows a sectional view of the inhaler 100 where it can be seen that in an initial state - upon the inhaler having been delivered to the user - the sharp inlet end of the channel 11 is not in contact with the septum of the medicament cartridge 2 being accommodated inside the main body 1. The loading button 8 is initially in the first position P1.

With reference to Figure 3b showing docking of the medicament cartridge 2; upon a user operating the loading button 8 in a direction towards a distal end of the main body 1 as indicated by the arrow, the sharp end of the channel 11 penetrates the septum of the cartridge 2 and thus moves into contact with the medicament contained therein.

In an embodiment, the spring loaded piston rod 4 is arranged with a gripping member 15 configured to move into a gripping engagement with the medicament cartridge 2 upon the loading button 8 initially being operated to move the spring loaded piston rod 4 into the second position P2 to cause the sharp inlet end of the channel 11 to penetrate the septum of the medicament cartridge 2.

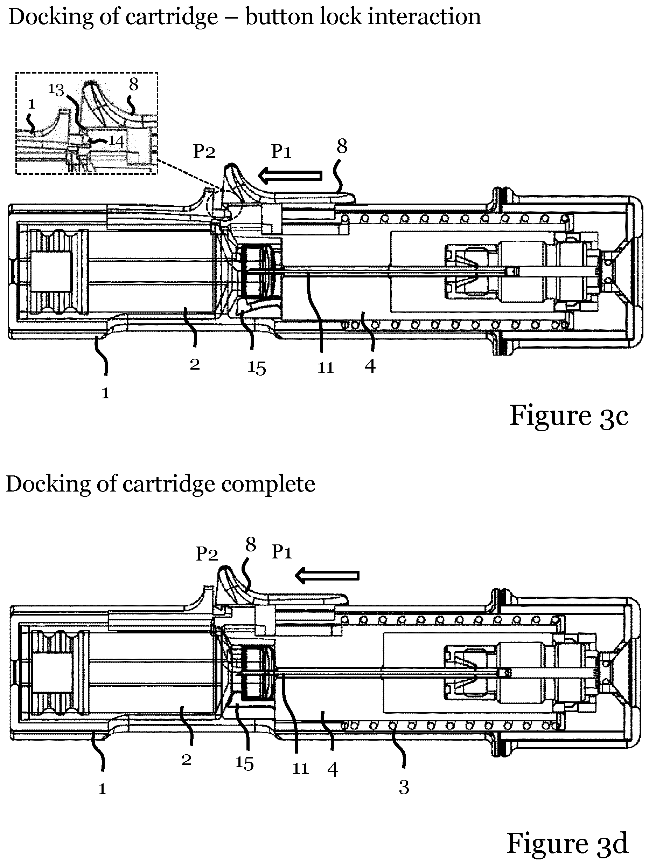

As the user moves the loading button 8 into position P2 illustrated in Figure 3c, the gripping member 15 moves into gripping engagement with the cartridge 2.

Further, in this embodiment, the spring loaded piston rod 4 is moved into locking engagement with the main body 1.

This may be achieved in an embodiment by the loading button 8 being arranged with a locking member 13 configured to move into engagement with a mating locking member 14 of the main body 1 for causing the locking engagement of the spring loaded piston rod 4 and the main body 1.

In an embodiment, the locking member 13 of the loading button 8 and the mating locking member 14 of the main body 1 are configured such that an audible indication is provided the user upon the two locking members 13, 14 moving into engagement, such as a clicking sound. Alternatively, or as a complement to the clicking sound, a tactile indication is provided the user upon the two locking members 13, 14 moving into engagement.

As can be seen in Figure 3c, in an embodiment, the locking member 13 of the loading button 8 is configured to cause a temporary deformation of the main body 1 (on the upper side of the body) upon moving into contact with the mating locking member 14 before the mating locking member 14 snaps into engagement with the locking member 13 of the loading button 8 to cause the audible and/or tactile indication.

Thus, with reference to Figure 3d, when the loading button 8 finally moves into position P2, the locking member 13 of the loading button 8 snaps into locking engagement with the mating locking member 14 of the main body 1 causing an audible and/or tactile indication to the user, while the gripping member 15 fully moves into gripping engagement with the cartridge 2 and the inlet end of the channel 11 moves further into the cartridge 2. At this stage, the spring loaded piston rod 4 is in locking engagement with the main body 1 and the cartridge 2 is in gripping engagement with the spring loaded piston rod 4 (and thus the channel 11). The docking is hence is complete.

Advantageously, since the user moves the loading button 8 into position P2 (and thus cannot move the loading button 8 any further in order to further compress the spring 3), the spring 3 may be selected to have an appropriate force to cause a pressure of 15-100 bar to be applied to the medicament transported to the mouthpiece 10 since a magnitude of that that pressure is solely controlled by the force of the spring 3 upon the loading button 8 being released from second end position P2.

The loading button 8 is in contact with the spring loaded piston rod 4 in the first position and thus pulls the piston rod 4 towards the distal end upon a user performing a loading movement thereby causing compression of the spring 3. However, upon the button 8 being operated such that the spring loaded piston rod 4 is released, the spring 3 returns to its expanded state and the rod 4 accordingly returns to the first position P1. The user is thus advantageously not capable of controlling degree of spring expansion by pushing the button 8 towards the proximal end of the inhaler 100; this is controlled solely by the spring 3 itself.

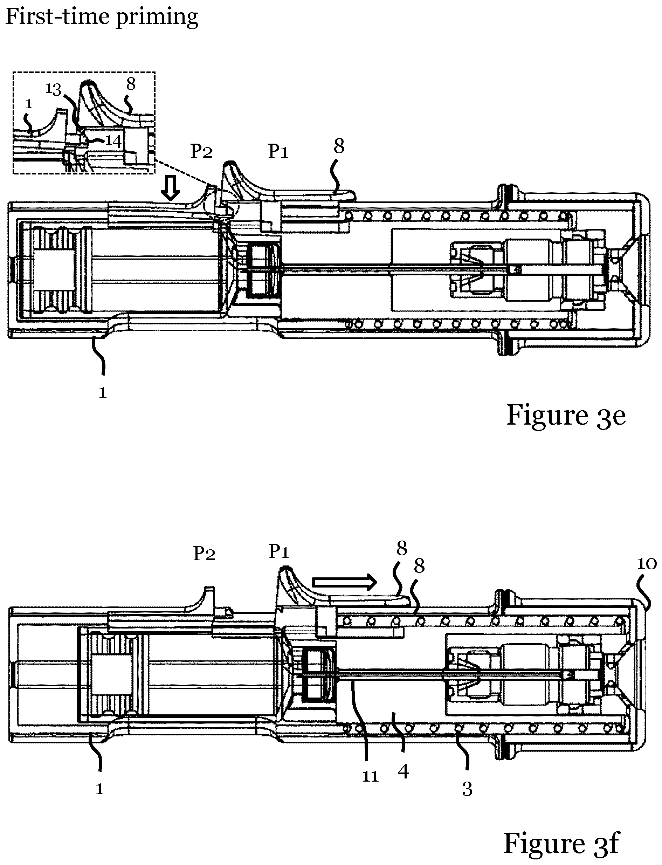

Figure 3e illustrates the user releasing the loading button 8 from position P2 to perform a first-time priming. In an embodiment, this is performed by the user pressing onto the main body 1 (as indicated by the vertically aligned arrow) which causes the mating locking member 14 of the main body 1 to snap out of engagement with the locking member 13 of the loading button 8 and release the spring loaded piston rod 4 from the locking engagement with the main body 1.

As shown in Figure 3f, the spring 3 will thus expand and move the spring loaded piston rod 4 into the first end position P1. A priming dose of medicament will thus be expelled from the cartridge 2. As is understood, before any priming have been performed, the channel 11 will be filled with air. Upon being expanded, the spring 3 will pressurize the medicament-air mix which is transported into the channel 11 (but which not necessarily is ejected from the inhaler 100 since the user will not inhale via the mouthpiece 10 at this stage). The inhaler 100 is now ready for use.

In another embodiment, where no locking engagement is to be attained between the spring loaded piston rod 4 and the main body 1, the user moves the locking button 8 into the second position P2 (thereby causing spring compression) and holds the locking button 8 in that position before releasing the locking button 8, wherein the spring 3 is expanded and causes the locking button 8 and the spring loaded piston rod 4 to return to the first position P1.

In such an embodiment, it may still be desired to provide an audible and/or tactile indication to the user that the button 8 (and thus the piston rod 4 moves into the second position).

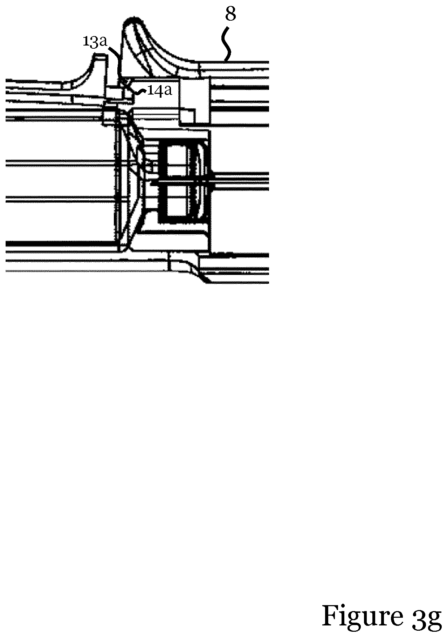

Figure 3g shows a detail of the inhaler where instead of using the previously described locking members 13, 14 causing locking engagement, the loading button 8 is arranged with a contacting member 13a configured to move into contact with a mating contacting member 14a of the main body 1, where the two contacting members 13a, 14a are configured to cause an audible and/or tactile indication to the user upon moving into contact. Thus, the contacting members 13a, 14a are arranged such that they do not cause a locking engagement, but nevertheless provide an audible and/or tactile indication to the user upon moving into contact.

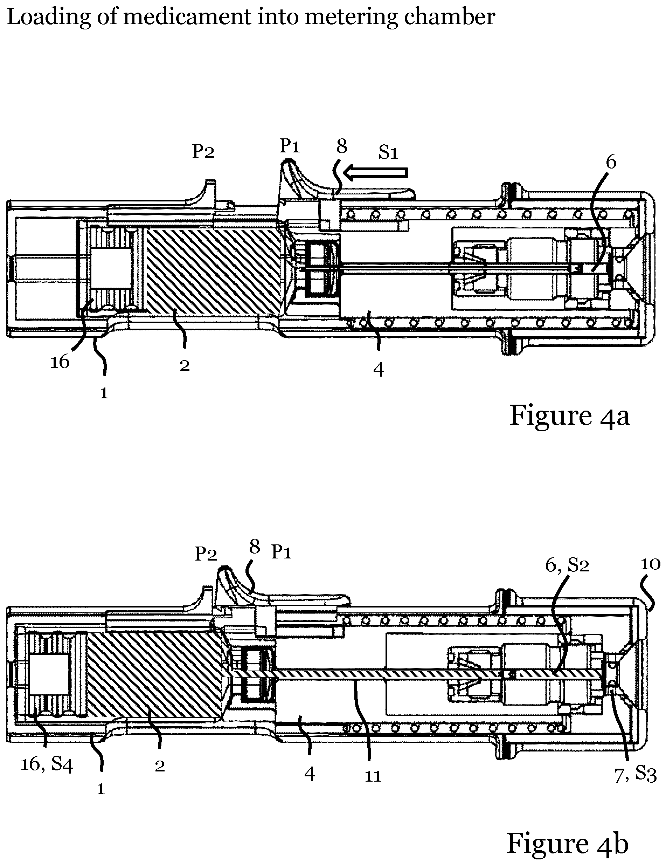

Figures 4a and 4b illustrate loading of medicament into the metering chamber 6 such that the user subsequently can inhale a dose of medicament. After the docking is complete and priming has been performed as was described with reference to Figure 3f, the loading button is in the first position P1.

In order to load the medicament (illustrated with a striped pattern) accommodated inside the cartridge 2 into the metering chamber 6, the user slides the loading button 8 towards the distal end of the main body 1, as indicated by S1.

This causes the spring loaded piston rod 4 to move into locking engagement with the main body 1 upon the loading button 8 being moved into the second position P2 and the locking members moving into locking engagement with each other as previously described.

This movement of the piston rod 4 creates underpressure in the metering chamber 6, and the medicament is transported from the cartridge 2 via the channel 11 to the metering chamber 6, as indicated by S2.

Further, as indicated at S3, high flow resistance in the MEMS nozzle 7 prevents air to be drawn through the nozzle 7.

In an embodiment, the medicament cartridge 2 comprises a low-friction plunger 16 movably attached to a bottom section the medicament cartridge 2, as indicated at S4 which is configured to move with the medicament along a longitudinal axis of inhaler 100 upon the medicament being transported from the medicament cartridge 2 via the channel 11 towards the metering chamber 6.

At this stage, the loading button 8 has been slided into position P2 by the user and the spring loaded piston rod 4 is in locking engagement with the main body 1. When the user operates the loading button 8, the user will slide the button 8 into the end position P2 and then release the button 8, whereby the button 8 and the piston rod 4 will move very slightly in a direction towards the proximal end of the inhaler where the locking member 13 of the loading button 8 locks into engagement with the mating locking member 14 of the main body 1 and thus stops further movement.

This very small movement of the spring loaded piston rod 4 in a direction towards the proximal end causes a small priming medicament dose to fill a cavity of the nozzle 7 thereby expelling any old medicament residues at the nozzle 7.

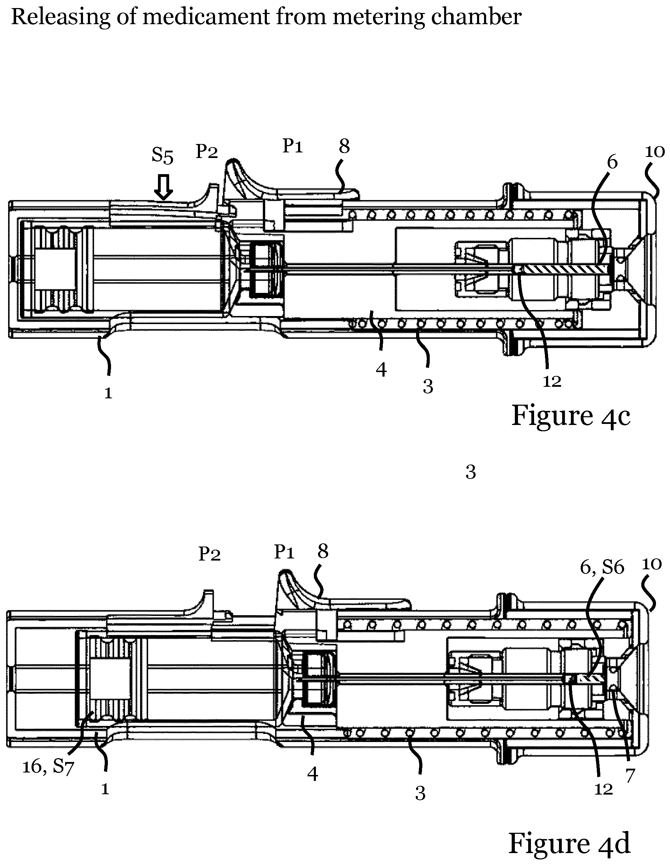

Finally, with reference to Figures 4c and 4d, the user releases a dose of medicament for inhalation via the mouthpiece 10.

At S5, the user operates the loading button 8 indirectly by pressing onto the main body 1 which causes the mating locking member 14 of the main body 1 to snap out of engagement with the locking member 13 of the loading button 8 and release the spring loaded piston rod 4 from the locking engagement with the main body 1. At the same time, the user inhales through the mouthpiece 10.

The spring 3 will thus expand and move the spring loaded piston rod 4 towards the first end position P1. As indicated at S6, the medicament in the metering chamber 6 is pressurized by the spring 3 and pushed through the membrane of the nozzle 7, which creates an aerosol being ejected by the inhaler 100 and inhaled by the user via the mouthpiece 10.

Further, the check valve 12 at the inlet end of the metering chamber 6 prevents medicament from flowing back into the channel 11.

Moreover, as the spring loaded piston rod 4 moves in the first position P1, the plunger 16 will move with the rod 4 as indicated at S7 to push the medicament in the cartridge 2 towards the outlet.

With this operation, a medicament dose has been delivered to the user.

The aspects of the present disclosure have mainly been described above with reference to a few embodiments and examples thereof. However, as is readily appreciated by a person skilled in the art, other embodiments than the ones disclosed above are equally possible within the scope of the invention, as defined by the appended patent claims.

Thus, while various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims.

## Claims

1. Medicament delivery device (100) configured to eject a dose of medicament for delivery to a user, comprising:
a main body (1) accommodating a medicament cartridge (2);
a piston rod (4) arranged at an outlet end of the medicament cartridge (2), the piston rod (4) accommodating a channel (11) an inlet end of which is arranged to be inserted in the medicament cartridge (2) for access to and transport of the medicament;
a spring (3) configured to spring load the piston rod (4) such that the spring (3) is compressed upon the piston rod (4) being moved towards a distal end of the main body (1);
a metering chamber (6) configured to accommodate a dose of medicament being transported via the piston rod channel (11);
a check valve (12) arranged at an inlet end of the metering chamber (4);
a medicament delivery member (10) arranged at an outlet end of the metering chamber (6) and configured to eject the dose of medicament in the metering chamber (6) for delivery to the user; and
a loading button (8) being configured to be accessible from an exterior of the main body (1)
which when operated by the user to move from a first position to a second position in a direction towards the distal end of the main body (1) contacts the spring loaded piston rod (4) and causes the spring loaded piston rod (4) to move towards said distal end and the spring (3) to compress, thereby causing the medicament to flow from the medicament cartridge (2) via the channel (11) into the metering chamber (6); and
which when operated by the user such that the spring loaded piston rod (4) is released causes the spring (3) to expand and the spring loaded piston rod (4) to move away from said distal end and into the first position, which movement causes the dose of medicament to leave the metering chamber (6) and flow through the medical delivery member (10) for delivery to the user.

2. The medicament delivery device (100) of claim 1, wherein:
the loading button (8) is configured to be slidably arranged along a longitudinal axis of the medicament delivery device (100) from the first position to the second position.

3. The medicament delivery device (100) of claims 1 or 2, wherein:
the spring loaded piston rod (4) is configured to move into locking engagement with the main body (1) upon the user operating the loading button (8) to move into the second position.

4. The medicament delivery device (100) of claim 3, wherein:
the loading button (8) is arranged with a locking member (13) configured to move into engagement with a mating locking member (14) of the main body (1) for causing the locking engagement of the spring loaded piston rod (4) and the main body (1).

5. The medicament delivery device (100) of claim 4, wherein:
the locking member (13) of the loading button (8) and the mating locking member (14) of the main body (1) are configured such that an audible indication is provided to the user upon the two locking members (13, 14) moving into engagement.

6. The medicament delivery device (100) of claims 4 or 5, wherein:
the locking member (13) of the loading button (8) and the mating locking member (14) of the main body (1) is configured such that a tactile indication is provided to the user upon the two locking members (13, 14) moving into engagement.

7. The medicament delivery device (100) of claims 5 or 6, wherein:
the locking member (13) of the loading button (4) is configured to cause a temporary deformation of the main body (1) upon moving into contact with said mating locking member (14) before the mating locking member (14) snaps into engagement with the locking member (13) of the loading button (8) to cause the audible and/or tactile indication.

8. The medicament delivery device (100) of claim 7, wherein:
the main body (1) is configured such that a user press onto the main body (1) causes the mating locking member (14) to snap out of engagement with the locking member (13) of the loading button (8) and release the spring loaded piston rod (4) from the locking engagement with the main body (1).

9. The medicament delivery device (100) of claims 1 or 2, wherein:
the loading button (8) is arranged with a contacting member (13a) configured to move into contact with a mating contacting member (14a) of the main body (1), the two contacting members (13a, 14a) being configured to cause an audible and/or tactile indication to the user upon moving into contact.

10. The medicament delivery device (100) of any one of the preceding claims,
wherein:
the spring loaded piston rod (4) is arranged with a gripping member (15) configured to move into a gripping engagement with the medicament cartridge (2) upon the loading button (8) initially being operated to move the spring loaded piston rod (4) into the second position to cause an inlet end of the channel (11) to penetrate a septum of the medicament cartridge (2).

11. The medicament delivery device (100) of any one of the preceding claims, further comprising:
an air flow shaper (9) arranged at an outlet end of the metering chamber (6).

12. The medicament delivery device (100) of any one of the preceding claims,
wherein:
the medicament cartridge (2) is arranged with a plunger (16) movably attached to a bottom section the medicament cartridge (2) which is configured to move with the medicament along a longitudinal axis of the medicament delivery device (100) upon the medicament being transported from the medicament cartridge (2) via the channel (11) towards the metering chamber (6).

13. The medicament delivery device (100) of any one of the preceding claims, further comprising:
a sealing member (5) arranged around the channel (11) in the spring loaded piston rod (4).

14. The medicament delivery device (100) of any one of the preceding claims, further comprising:
a nozzle (7) arranged at an outlet end of the metering chamber (6) and comprising a membrane through which the medicament passes and which is configured to eject the medicament via the medicament delivery member (10) as an aerosol for inhalation by the user.

15. The medicament delivery device (100) of any one of the preceding claims,
wherein:
the medicament indicator (17) comprises a window (18) configured to allow the user to view the medicament in the medicament cartridge (2).

## Patentansprüche

1. Medikamentenabgabevorrichtung (100), die konfiguriert ist, um eine Medikamentendosis für eine Abgabe an einen Benutzer auszustoßen, umfassend:
einen Hauptkörper (1), der eine Medikamentenpatrone (2) aufnimmt;
eine Kolbenstange (4), die an einem Auslassende der Medikamentenpatrone (2) angeordnet ist, wobei die Kolbenstange (4) einen Kanal (11) aufnimmt, dessen Einlassende angeordnet ist, um in die Medikamentenpatrone (2) für einen Zugriff auf das Medikament und einen Transport davon eingeführt zu werden;
eine Feder (3), die konfiguriert ist, um die Kolbenstange (4) derart zu federn, dass die Feder (3) zusammengedrückt wird, wenn die Kolbenstange (4) zu einem distalen Ende des Hauptkörpers (1) hin bewegt wird;
eine Dosierungskammer (6), die konfiguriert ist, um eine Medikamentendosis aufzunehmen, die über den Kolbenstangenkanal (11) transportiert wird;
ein Rückschlagventil (12), das an einem Einlassende der Dosierungskammer (4) angeordnet ist;
ein Medikamentenabgabeelement (10), das an einem Auslassende der Dosierungskammer (6) angeordnet und konfiguriert ist, um die Medikamentendosis in der Dosierungskammer (6) für die Abgabe an den Benutzer auszustoßen; und
einen Ladeknopf (8), der konfiguriert ist, um von einer Außenseite des Hauptkörpers (1) aus zugänglich zu sein
der, wenn durch den Benutzer betätigt, um sich von einer ersten Position in eine zweite Position in einer Richtung zu dem distalen Ende des Hauptkörpers (1) hin zu bewegen, die federbelastete Kolbenstange (4) berührt und bewirkt, dass sich die federbelastete Kolbenstange (4) zu dem distalen Ende hin bewegt und die Feder (3) zusammengedrückt wird, wodurch bewirkt wird, dass das Medikament aus der Medikamentenpatrone (2) über den Kanal (11) in die Dosierungskammer (6) fließt; und
der, wenn durch den Benutzer derart betätigt, dass die federbelastete Kolbenstange (4) freigegeben wird, bewirkt, dass sich die Feder (3) ausdehnt und sich die federbelastete Kolbenstange (4) von dem distalen Ende weg und in die erste Position bewegt, wobei die Bewegung bewirkt, dass die Medikamentendosis die Dosierungskammer (6) verlässt und durch das medizinische Abgabeelement (10) fließt, für die Abgabe an den Benutzer.

2. Medikamentenabgabevorrichtung (100) nach Anspruch 1, wobei:
der Ladeknopf (8) konfiguriert ist, um entlang einer Längsachse der Medikamentenabgabevorrichtung (100) von der ersten Position in die zweite Position verschiebbar angeordnet zu sein.

3. Medikamentenabgabevorrichtung (100) nach Anspruch 1 oder 2, wobei:
die federbelastete Kolbenstange (4) konfiguriert ist, um sich in Verriegelungseingriff mit dem Hauptkörper (1) zu bewegen, wenn der Benutzer den Ladeknopf (8) betätigt, um sich in die zweite Position zu bewegen.

4. Medikamentenabgabevorrichtung (100) nach Anspruch 3, wobei:
der Ladeknopf (8) mit einem Verriegelungselement (13) angeordnet ist, das konfiguriert ist, um mit einem zusammenpassenden Verriegelungselement (14) des Hauptkörpers (1) in Eingriff zu treten, um den Verriegelungseingriff zwischen der federbelasteten Kolbenstange (4) und dem Hauptkörper (1) zu bewirken.

5. Medikamentenabgabevorrichtung (100) nach Anspruch 4, wobei:
das Verriegelungselement (13) des Ladeknopfs (8) und das zusammenpassende Verriegelungselement (14) des Hauptkörpers (1) derart konfiguriert sind, dass dem Benutzer eine hörbare Anzeige bereitgestellt wird, wenn die zwei Verriegelungselemente (13, 14) in Eingriff treten.

6. Medikamentenabgabevorrichtung (100) nach Anspruch 4 oder 5, wobei:
das Verriegelungselement (13) des Ladeknopfs (8) und das zusammenpassende Verriegelungselement (14) des Hauptkörpers (1) derart konfiguriert sind, dass dem Benutzer eine fühlbare Anzeige bereitgestellt wird, wenn die zwei Verriegelungselemente (13, 14) in Eingriff treten.

7. Medikamentenabgabevorrichtung (100) nach Anspruch 5 oder 6, wobei:
das Verriegelungselement (13) des Ladeknopfs (4) konfiguriert ist, um eine vorübergehende Verformung des Hauptkörpers (1) zu verursachen, wenn er in Berührung mit dem zusammenpassenden Verriegelungselement (14) kommt, bevor das zusammenpassende Verriegelungselement (14) in Eingriff mit dem Verriegelungselement (13) des Ladeknopfs (8) einschnappt, um die hörbare und/oder fühlbare Anzeige zu bewirken.

8. Medikamentenabgabevorrichtung (100) nach Anspruch 7, wobei:
der Hauptkörper (1) derart konfiguriert ist, dass ein Druck des Benutzers auf den Hauptkörper (1) bewirkt, dass das zusammenpassende Verriegelungselement (14) aus dem Eingriff mit dem Verriegelungselement (13) des Ladeknopfs (8) schnappt und die federbelastete Kolbenstange (4) aus dem Verriegelungseingriff mit dem Hauptkörper (1) freigibt.

9. Medikamentenabgabevorrichtung (100) nach Anspruch 1 oder 2, wobei:
der Ladeknopf (8) mit einem Berührungselement (13a) angeordnet ist, das konfiguriert ist, um mit einem zusammenpassenden Berührungselement (14a) des Hauptkörpers (1) in Berührung zu kommen, wobei die zwei Berührungselemente (13a, 14a) konfiguriert sind, um bei dem Inberührungkommen eine hörbare und/oder fühlbare Anzeige für den Benutzer zu bewirken.

10. Medikamentenabgabevorrichtung (100) nach einem der vorstehenden Ansprüche, wobei:
die federbelastete Kolbenstange (4) mit einem Greifelement (15) angeordnet ist, das konfiguriert ist, um sich in einen Greifeingriff mit der Medikamentenpatrone (2) zu bewegen, wenn der Ladeknopf (8) zunächst betätigt wird, um die federbelastete Kolbenstange (4) in die zweite Position zu bewegen, um zu bewirken, dass ein Einlassende des Kanals (11) ein Septum der Medikamentenpatrone (2) durchdringt.

11. Medikamentenabgabevorrichtung (100) nach einem der vorstehenden Ansprüche, ferner umfassend:
einen Luftstromformer (9), der an einem Auslassende der Dosierungskammer (6) angeordnet ist.

12. Medikamentenabgabevorrichtung (100) nach einem der vorstehenden Ansprüche, wobei:
die Medikamentenpatrone (2) mit einem Kolben (16) angeordnet ist, der bewegbar an einem unteren Abschnitt der Medikamentenpatrone (2) angebracht und konfiguriert ist, um sich mit dem Medikament entlang einer Längsachse der Medikamentenabgabevorrichtung (100) zu bewegen, wenn das Medikament von der Medikamentenpatrone (2) über den Kanal (11) zu der Dosierungskammer (6) hin transportiert wird.

13. Medikamentenabgabevorrichtung (100) nach einem der vorstehenden Ansprüche, ferner umfassend:
ein Dichtungselement (5), das um den Kanal (11) herum in der federbelasteten Kolbenstange (4) angeordnet ist.

14. Medikamentenabgabevorrichtung (100) nach einem der vorstehenden Ansprüche, ferner umfassend:
eine Düse (7), die an einem Auslassende der Dosierkammer (6) angeordnet ist und umfassend eine Membran, durch die das Medikament hindurchtritt, und die konfiguriert ist, um das Medikament über das Medikamentenabgabeelement (10) als ein Aerosol für eine Inhalation durch den Benutzer auszustoßen.

15. Medikamentenabgabevorrichtung (100) nach einem der vorstehenden Ansprüche, wobei:
die Medikamentenanzeige (17) ein Fenster (18) umfasst, das konfiguriert ist, um dem Benutzer zu ermöglichen, das Medikament in der Medikamentenpatrone (2) zu sehen.

## Revendications

1. Dispositif d'administration de médicament (100) configuré pour éjecter une dose de médicament pour l'administration à un utilisateur, comprenant :
un corps principal (1) recevant une cartouche de médicament (2) ;
une tige de piston (4) disposée au niveau d'une extrémité de sortie de la cartouche de médicament (2), la tige de piston (4) recevant un canal (11) dont une extrémité d'entrée est conçue pour être insérée dans la cartouche de médicament (2) afin d'accéder au médicament et de le transporter ;
un ressort (3) conçu pour charger la tige de piston (4) de telle sorte que le ressort (3) est comprimé lorsque la tige de piston (4) est déplacée vers une extrémité distale du corps principal (1) ;
une chambre de dosage (6) conçue pour recevoir une dose de médicament transportée par le canal de tige de piston (11) ;
un clapet anti-retour (12) disposé au niveau d'une extrémité d'entrée de la chambre de dosage (4) ;
un élément d'administration de médicament (10) disposé au niveau d'une extrémité de sortie de la chambre de dosage (6) et configuré pour éjecter la dose de médicament dans la chambre de dosage (6) pour l'administration à l'utilisateur ; et
un bouton de chargement (8) conçu pour être accessible depuis une partie extérieure du corps principal (1)
qui, lorsqu'il est actionné par l'utilisateur pour passer d'une première position à une seconde position en direction de l'extrémité distale du corps principal (1), entre en contact avec la tige de piston à ressort (4) et provoque le déplacement de la tige de piston à ressort (4) vers ladite extrémité distale et la compression du ressort (3), provoquant ainsi l'écoulement du médicament de la cartouche de médicament (2) par le canal (11) dans la chambre de dosage (6) ; et
qui, lorsqu'il est actionné par l'utilisateur de telle sorte que la tige de piston à ressort (4) est relâchée, provoque l'expansion du ressort (3) et l'éloignement de la tige de piston à ressort (4) de ladite extrémité distale et son arrivée dans la première position, lequel mouvement amène la dose de médicament à quitter la chambre de dosage (6) et à s'écouler à travers l'élément d'administration de médicament (10) pour l'administration à l'utilisateur.

2. Dispositif d'administration de médicament (100) selon la revendication 1,
dans lequel :
le bouton de chargement (8) est conçu pour être disposé de manière coulissante le long d'un axe longitudinal du dispositif d'administration de médicament (100), de la première position à la seconde position.

3. Dispositif d'administration de médicament (100) selon les revendications 1 ou 2, dans lequel :
la tige de piston à ressort (4) est conçue pour venir en prise avec verrouillage avec le corps principal (1) lorsque l'utilisateur actionne le bouton de chargement (8) pour le déplacer dans la seconde position.

4. Dispositif d'administration de médicament (100) selon la revendication 3,
dans lequel :
le bouton de chargement (8) est doté d'un élément de verrouillage (13) conçu pour venir en prise avec un élément de verrouillage homologue (14) du corps principal (1) afin de provoquer la mise en prise avec verrouillage de la tige de piston à ressort (4) et du corps principal (1).

5. Dispositif d'administration de médicament (100) selon la revendication 4,
dans lequel :
l'élément de verrouillage (13) du bouton de chargement (8) et l'élément de verrouillage homologue (14) du corps principal (1) sont conçus de telle sorte qu'une indication sonore est fournie à l'utilisateur lorsque les deux éléments de verrouillage (13, 14) viennent en prise.

6. Dispositif d'administration de médicament (100) selon les revendications 4 ou 5, dans lequel :
l'élément de verrouillage (13) du bouton de chargement (8) et l'élément de verrouillage homologue (14) du corps principal (1) sont conçus de telle sorte qu'une indication tactile est fournie à l'utilisateur lorsque les deux éléments de verrouillage (13, 14) viennent en prise.

7. Dispositif d'administration de médicament (100) selon les revendications 5 ou 6, dans lequel :
l'élément de verrouillage (13) du bouton de chargement (4) est conçu pour provoquer une déformation temporaire du corps principal (1) lorsqu'il vient en contact avec ledit élément de verrouillage homologue (14) avant que l'élément de verrouillage homologue (14) ne s'enclenche en prise avec l'élément de verrouillage (13) du bouton de chargement (8) pour provoquer l'indication sonore et/ou tactile.

8. Dispositif d'administration de médicament (100) selon la revendication 7,
dans lequel :
le corps principal (1) est conçu de telle sorte qu'une pression de l'utilisateur sur le corps principal (1) fait sortir l'élément de verrouillage (14) de sa mise en prise avec l'élément de verrouillage (13) du bouton de chargement (8) et libère la tige de piston à ressort (4) de la mise en prise avec verrouillage avec le corps principal (1).

9. Dispositif d'administration de médicament (100) selon les revendications 1 ou 2, dans lequel :
le bouton de chargement (8) est doté d'un élément de contact (13a) conçu pour venir en contact avec un élément de contact homologue (14a) du corps principal (1), les deux éléments de contact (13a, 14a) étant conçus pour provoquer une indication sonore et/ou tactile à l'utilisateur lorsqu'ils viennent en contact.

10. Dispositif d'administration de médicament (100) selon l'une quelconque des revendications précédentes, dans lequel :
la tige de piston à ressort (4) est dotée d'un élément de préhension (15) conçu pour venir en prise par préhension avec la cartouche de médicament (2) lorsque le bouton de chargement (8) est initialement actionné pour déplacer la tige de piston à ressort (4) dans la seconde position afin de faire pénétrer une extrémité d'entrée du canal (11) dans un septum de la cartouche de médicament (2).

11. Dispositif d'administration de médicament (100) selon l'une quelconque des revendications précédentes, comprenant en outre :
un dispositif de mise en forme de flux d'air (9) disposé au niveau d'une extrémité de sortie de la chambre de dosage (6).

12. Dispositif d'administration de médicament (100) selon l'une quelconque des revendications précédentes, dans lequel :
la cartouche de médicament (2) est dotée d'un piston (16) fixé de manière mobile à une section inférieure de la cartouche de médicament (2) qui est conçue pour se déplacer avec le médicament le long d'un axe longitudinal du dispositif d'administration de médicament (100) lors du transport du médicament de la cartouche de médicament (2) par le canal (11) vers la chambre de dosage (6).

13. Dispositif d'administration de médicament (100) selon l'une quelconque des revendications précédentes, comprenant en outre :
un élément d'étanchéité (5) disposé autour du canal (11) dans la tige de piston à ressort (4).

14. Dispositif d'administration de médicament (100) selon l'une quelconque des revendications précédentes, comprenant en outre :
une buse (7) disposée au niveau d'une extrémité de sortie de la chambre de dosage (6) et comprenant une membrane à travers laquelle passe le médicament, et qui est conçue pour éjecter le médicament par l'intermédiaire de l'élément d'administration de médicament (10) sous forme d'un aérosol pour l'inhalation par l'utilisateur.

15. Dispositif d'administration de médicament (100) selon l'une quelconque des revendications précédentes, dans lequel :
l'indicateur de médicament (17) comprend une fenêtre (18) conçue pour permettre à l'utilisateur de voir le médicament dans la cartouche de médicament (2).
